# EUROPEAN PATENT SPECIFICATION

(11) **EP 4 099 942 B1**
(45) Date of publication and mention of the grant of the patent: **11.11.2026**
(21) Application number: 21724398.9
(22) Date of filing: 16.03.2021
(51) Int. Cl.: A61B 90/00, A61B 17/00, A61B 10/02

(54) **NON-MIGRATING BIOPSY SITE IDENTIFIERS**
NICHT MIGRIERENDE IDENTIFIKATOREN FÜR BIOPSIESTELLEN
IDENTIFICATEURS DE SITES DE BIOPSIE NON MIGRANTS

(30) Priority: 17.03.2020 US 202062990571 P
(43) Date of publication of application: 14.12.2022
(73) Proprietor: Devicor Medical Products, Inc., Cincinnati, OH 45241 (US)
(72) Inventor: NICHOLAS, Emily, West Chester, OH 45069 (US); SANDER, Dave, Lebanon, OH 45036 (US); TANGHAL, Emmanuel, V., Mason, OH 45040 (US); KELLER, Bryan, R., Loveland, OH 45140 (US); CALLAHAN, Sean, Liberty Township, OH 45044 (US); MCCLURE, Jeffrey, Lebanon, OH 45036 (US)
(74) Representative: Simmons & Simmons
(86) International application number: PCT/US2021/022473
(87) International publication number: WO 2021/188479

(56) References cited:
- WO-A1-00/24332
- WO-A1-01/08578
- WO-A1-2016/073912
- WO-A1-96/08208
- WO-A2-00/38579
- US-A1- 2005 119 562
- US-A1- 2012 226 146
- US-A1- 2014 018 663

## Description

### BACKGROUND

A number of patients will have breast biopsies because of irregular mammograms and palpable abnormalities. Biopsies can include surgical excisional biopsies and stereotactic and ultrasound guided needle breast biopsies. In the case of image directed biopsy, the radiologist or other physician may take a small sample of the irregular tissue for laboratory analysis. If the biopsy proves to be malignant, additional surgery (e.g., a lumpectomy or a mastectomy) may be required. In the case of needle biopsies, the patient may return to the radiologist a day or more later, and the biopsy site (the site of the lesion) may need to be relocated in preparation for the surgery. An imaging system, such as ultrasound, magnetic resonance imaging (MRI) or x-ray may be used to locate the biopsy site. In order to assist the relocation of the biopsy site, a marker may be placed at the time of the biopsy.

The use of markers used after breast biopsies to mark the location where the biopsied tissue was removed is described in the following US Patents: US 6,083,524, "Polymerizable biodegradable polymers including carbonate or dioxanone linkages," issued July 4, 2000; US 6,162,241, "Hemostatic tissue sealants," issued December 4, 2000; US 6,270,464, "Biopsy localization method and device," issued August 7, 2001; US 6,356,782, "Subcutaneous cavity marking device and method," issued March 12, 2002; US 6,605,294, "Methods of using in situ hydration of hydrogel articles for sealing or augmentation of tissue or vessels," issued August 12, 2003; US 8,600,481, "Subcutaneous cavity marking device," issued December 3, 2013 and US 8,939,910, "Method for enhancing ultrasound visibility of hyperechoic materials", issued January 27, 2015.

Once a marker is placed at a biopsy site, the marker can later be relocated to identify the biopsy site in subsequent follow-up procedures. In some contexts, a placed marker may not completely correspond to the biopsy site when the marker is relocated. For instance, the marker may migrate to another nearby location during the intervening time between the biopsy procedure and subsequent follow up procedures. Migration of the biopsy site marker can cause difficulties when identifying the biopsy site during subsequent follow-up procedures. Accordingly, it may be desirable to incorporate features into a marker to maintain the marker in a fixed position over time.

While several systems and methods have been made and used for marking a biopsy site, it is believed that no one prior to the inventor has made or used the invention described in the appended claims.
WO 96/082208 discloses a range of expanding marker elements for deployment from a tube of a deployment instrument.
WO01/08578 discloses helical markers for large-bore and open biopsy procedures.
US2012/0226146 discloses biopsy markers with opposed hooks joined by a resilient section to retain the marker at the tissue site.

### SUMMARY

The invention is defined by the independent claim. A selection of optional features is set out in the dependent claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements. In the drawings some components or portions of components are shown in phantom as depicted by broken lines.
FIGS. 1A, 1B, and 1C show exemplary aspects of placement of a biopsy site marker, in accordance with aspects of the present disclosure;
FIG. 2 depicts a perspective view of an exemplary marker delivery device;
FIG. 3 depicts a side cross-sectional view of the marker delivery device of FIG. 2;
FIG. 4 depicts a cross-sectional view of a marker being deployed from the distal portion of the marker delivery device of FIG. 2 and through a lateral aperture in a biopsy needle to mark a biopsy site;
FIG. 5A depicts a perspective view of another exemplary biopsy site marker;
FIG. 5B depicts a front cross-sectional view of the marker of FIG. 5A inside of a marker delivery device;
FIG. 5C depicts a cross-sectional view of the marker of FIG. 5A deployed inside of a cavity;
FIG. 6A depicts a side elevational view of yet another exemplary biopsy marker, with the marker in an initially expanded state;
FIG. 6B depicts a cross-sectional view of the marker of FIG. 6A, with the marker in a retracted state;
FIG. 7A depicts a perspective view of still another exemplary biopsy marker;
FIG. 7B depicts a side elevational view of a tab of the marker of FIG. 7A;
FIG. 7C depicts a side elevational view of still another exemplary biopsy marker;
FIG. 8A depicts a perspective view of still another exemplary biopsy marker;
FIG. 8B depicts a cross-sectional view of the marker of FIG. 8A, with the marker having a substantial triangular cross-section;
FIG. 8C depicts a cross-sectional view of still another exemplary biopsy marker having an alternative configuration of the marker of FIG. 8A;
FIG. 9 depicts a perspective view of still another exemplary biopsy marker;
FIG. 10A depicts a side elevational view of still another exemplary biopsy marker;
FIG. 10B depicts a side elevational view of the marker of FIG. 10A, with an alternative profile;
FIG. 11A depicts a side elevational view of still another exemplary biopsy marker, with the marker in a pre-deployment state;
FIG. 11B depicts a side elevational view of the marker of FIG. 11A, with the marker in a deployed state;
FIG. 12A depicts a side elevational view of still another exemplary biopsy marker, with the marker in a closed state;
FIG. 12B depicts a side elevational view of the marker of FIG. 12A, with the marker in an open state;
FIG. 13 depicts a side elevational view of still another exemplary biopsy marker;
FIG. 14A depicts a perspective view of still another exemplary biopsy marker, with the marker in an unrolled state; and
FIG. 14B depicts a side elevational view of the marker of FIG. 14A, with the marker in a rolled state.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It may be beneficial to be able to mark the location or margins of a lesion, whether temporarily or permanently, prior to or immediately after removing or sampling it. Marking prior to removal may help to ensure that the entire lesion is excised, if desired. Alternatively, if the lesion were inadvertently removed in its entirety, marking the biopsy site immediately after the procedure would enable reestablishment of its location for future identification.

Once a marker is positioned at a biopsy site, it may be desirable for the marker to remain visible under ultrasound. It may also be desirable to make the marker readily identifiable relative to other structural features of a patient. For instance, it may be desirable for the marker to be distinguishable under ultrasound visualization from microcalcifications to avoid inadvertently characterizing the marker as a microcalcification during subsequent ultrasonic examinations. Generally, microcalcifications are used in the field to identify suspicious lesions or masses. Thus, it is generally desirable for the ultrasound view to be distinguishable as a marker and not inadvertently identified as a new mass.

### I. Exemplary Marker

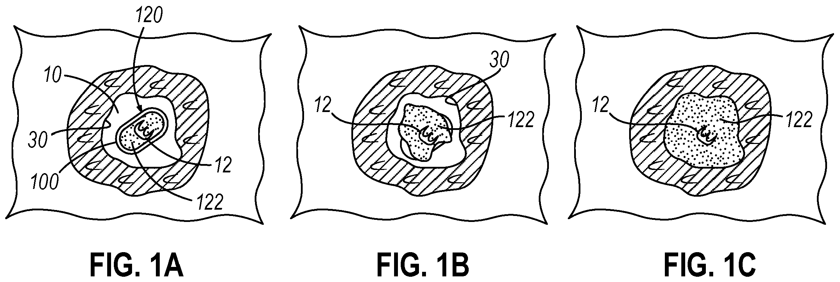

Aspects presented herein relate to devices and procedures for manufacturing a marker for percutaneously marking a biopsy cavity (10) having surrounding tissue (30), as shown in FIGS. 1A-1C. For instance, as seen in FIG. 1A, a marker (100) may be initially placed in the biopsy cavity (10) to facilitate relocation of the biopsy site. Marker (100) may comprise a carrier (120) and a marker element (12). Carrier (120) generally includes a bioabsorbable marker material (122). Thus, carrier (120) is generally configured for absorption into a patient after placement of marker (100) within the biopsy cavity (10). In some examples, carrier (120) can include a plurality of microbubbles to enhance visualization of carrier (120) under ultrasound. As will be described in greater detail below, marker material (122) is generally bioabsorbable such that marker material (122) may be generally absorbed into the patient's tissue over time. In the present example, marker material (122) comprises a hydrogel that is initially in a dehydrated state. Although a hydrogel is used in the present example, it should be understood that in other examples marker material (122) may comprise other known bioabsorbable materials

In the present example, marker (100) further includes a marker element (12) that is generally not bioabsorbable. Marker element (12) may comprise a radiopaque or echogenic marker embedded within the bioabsorbable marker material (122) of carrier (120). For instance, marker element (12) may comprise metal, hard plastic, or other radiopaque or hyperechoic materials known to those of ordinary skill in the art in view of the teachings herein.

Marker material (122) is generally expandable once disposed within a patient at a biopsy site. As shown in FIGS. 1B and 1C, the initially dehydrated marker material (122) may absorb fluid from the surrounding tissue (30) into which it is inserted. In response to this absorption of fluid, maker material (122) may swell, thereby permitting carrier (120) to fill a cavity formed at a biopsy site by removal of tissue samples during a biopsy procedure. Biodegradable materials may be particularly suitable in applications where it is desired that natural tissue growth be permitted to completely or partially replace the implanted material over time. Accordingly, biocompatibility is ensured and the natural mechanical parameters of the tissue are substantially restored to those of the pre-damaged condition.

Marker (100) may be inserted into the body either surgically via an opening in the body cavity (30), or through a minimally invasive procedure using such devices as a catheter, introducer or similar type insertion device. Marker (100) may be delivered immediately after removal of the tissue specimen using the same device used to remove the tissue specimen itself. Follow-up noninvasive detection techniques, such as x-ray mammography or ultrasound may then be used by the physician to identify, locate, and monitor the biopsy cavity site over a period of time via marker (100).

Marker (100) of the present example is large enough to be readily visible to a clinician under x-ray or ultrasonic viewing, for example; yet small enough to be able to be percutaneously deployed into the biopsy cavity and to not cause any difficulties with the patient. Although examples are described in connection with treatment and diagnosis of breast tissue, aspects presented herein may be used for markers in any internal, tissue, e.g., in breast tissue, lung tissue, prostate tissue, lymph gland tissue, etc.

The hydration of the marker material (122) of carrier (120) by the natural moisture of the tissue surrounding it causes expansion of the polymer and thus minimizes the risk of migration. The growing hydrogel-based marker material (122) centers marker (100) in the biopsy cavity as it grows. As the hydrogel expands, naturally present moisture from the surrounding tissue, the hydration enables increasing sound through transmission, appears more and more hypoechoic and is easy to visualize on follow up ultrasound studies.

The hydrated hydrogel marker material (122) of carrier (120) may also be used to frame permanent marker (12). The hypoechoic nature of the hydrated marker material (122) enables ultrasound visibility of the permanent marker (12) within the hydrogel hydrated marker material (122) because the permanent marker (12) is outlined as a specular reflector within a hypoechoic hydrated marker having a water-like nonreflective substrate.

### II. Exemplary Marker Delivery Device

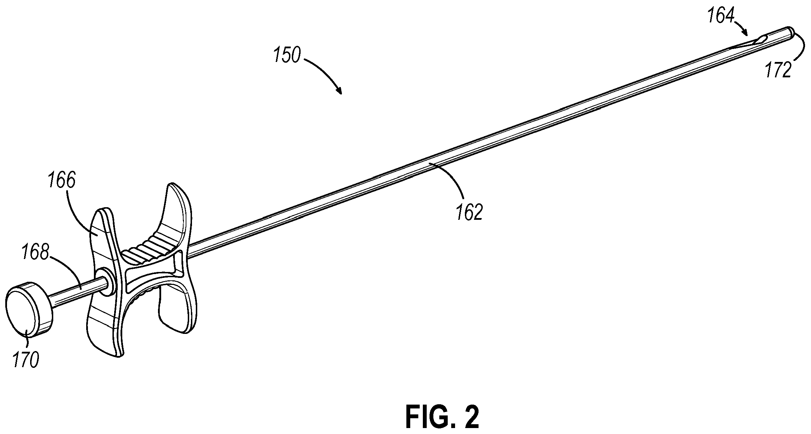

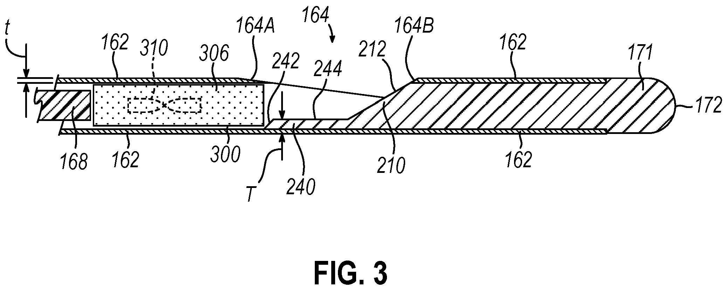

In some examples it may be desirable to deploy marker (100) described above within the body cavity (30) using certain marker delivery devices. For instance, FIGS. 2 and 3 show an exemplary marker delivery device (150) which includes an elongate outer cannula (162) having a marker exit, such as side opening (164) formed adjacent to, but spaced proximally from, the distal end of the cannula (162).

A grip (166) can be provided at the proximal end of cannula (162). A push rod (168) can be provided, with push rod (168) extending coaxially in cannula (162) such that push rod (168) is configured to translate within cannula (162) to displace one or more markers through side opening (164) (see FIG. 3). Rod (168) may have sufficient rigidity in compression to push a marker from an internal lumen (165) of cannula (162) out through opening (164), yet be relatively flexible in bending. A plunger (170) is coupled at the proximal end of rod (168) for forcing rod (168) distally in cannula (162) to deploy a marker out of cannula (162).

A user may grasp grip (166) with two fingers, and may push on plunger (170) using the thumb on the same hand, so that marker delivery device (160) is operated by a user's single hand. A spring (not shown) or other feature may be provided about rod (168) to bias rod (168) proximally relative to grip (166) and cannula (162).

FIG. 3 shows a cross-sectional view of a distal portion of the marker delivery device (160). As can be seen, a biopsy marker (300) similar to marker (100) described above is disposed within internal lumen (165) of cannula (162). In the present example, marker (300) comprise a biodegradable or otherwise resorbable marker material (306), such as a generally cylindrically shaped body of collagen, hydrogel, or etc., and a metallic, generally radiopaque permanent marker or marker element (310) (shown in phantom) disposed within or otherwise carried by marker material (306).

Cannula (162) may be formed of any suitable metallic or non-metallic material. In some versions, cannula (162) is formed of a thin walled hollow tube formed of a suitable medical grade plastic or polymer. One suitable material is a thermoplastic elastomer, such as Polyether block amide (PEBA), such as is known under the tradename PEBAX. Cannula (162) may be formed of PEBAX, and may be substantially transparent to visible light and X-ray.

Side opening (164) may be formed by cutting away a portion of the wall of cannula (162). Side opening (164) communicates with an internal lumen (165) of cannula (162). Side opening (164) may extend axially (in a direction parallel to the axis of lumen (165)) from a proximal opening end (164A) to a distal opening end (164B), as illustrated in FIG. 3.

In the present example, distal tip (172) extends from the distal end of cannula (162) and is rounded as shown in FIG. 3. Referring to FIG. 3, the distal end of cannula (162) is closed by a unitary endpiece (171), with a portion of endpiece (171) extending into internal lumen (165) of cannula (162). Endpiece (171) may be a molded or cast component. Endpiece (171) comprises a tip (172), a ramp (210) having a ramp surface (212), and a marker engaging element (240). Ramp surface (212) aids in directing marker (300) from internal lumen (165) through side opening (164). Marker engaging element (240) helps to retain marker (300) in internal lumen (165) until the user intends to deploy marker (300).

Marker engaging element (240) is disposed within internal lumen (165), and at least a portion of marker engaging element (240) is disposed distally of proximal end (164A) of side opening (164). Marker engaging element (240) extends along a portion of the floor of cannula (162) under opening (164) such that marker engaging element (240) is positioned to reinforce the portion of cannula (162) in which opening (164) is formed. For instance, by positioning marker engaging element (240) underneath opening (164), as shown in FIG. 3, element (240) helps to stiffen cannula (162) in the region where wall of cannula (162) is cut to form opening (164). As shown in FIG. 3, marker engaging element (240) extends from the proximal most portion of ramp surface (212), and does not extend proximally of side opening (164), though in other embodiments, a portion of element (240) may extend proximally of opening (164).

As shown in FIG. 3, marker engaging element (240) is in the form of a step having a generally uniform thickness (T) along element's (240) axial length, except that element (240) has a tapered proximal end (242). Tapered proximal end (242) forms an included angle with the longitudinal axis of lumen (165) (included angle with a horizontal line in FIG. 3) of about 45 degrees, while ramp surface (212) forms an included angle with the longitudinal axis of about 30 degrees. Of course, any number of other suitable angles may be used.

As shown in FIG. 3, an upwardly facing surface (244) (surface facing opening (164)) of marker engaging element (240) extends distally to contact ramp surface (212), so that there is not a space or gap between surface (244) and ramp surface (212). Such an arrangement is advantageous to reduce the possibility that marker (300), upon moving past marker engaging element (240), may become lodged between marker engagement element (240) and ramp (212). In some versions, marker engaging element (240), ramp (210), and/or tip (172) are formed of, or include, a material that is relatively more radiopaque than the wall of cannula (162). For instance, where element (240), ramp (210), and tip (172) are formed as an integral endpiece (171), endpiece (171) may include a radiopaque additive, such as barium sulfate. For instance, endpiece (171) may be a component molded of PEBAX, with about 20 percent by weight barium sulfate added to the molten PEBAX mold composition. The relatively more radiopaque marker engaging element (240), ramp (210), and tip (22) may be useful in distinguishing the position of those components using radiographic imaging. Also, where ramp (210) and/or step of engaging element (240) are positioned in association with opening (164), the addition of a radiopaque material can help identify the position of opening (164), and the position of marker (300) relative to opening (164) before, during, or after deployment of marker (300).

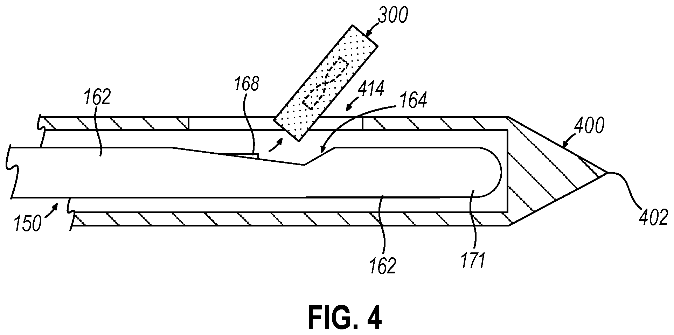

Referring to FIG. 4, marker delivery device (160) is used to deploy a marker (300) to mark a biopsy location within a patient. In FIG. 4, a cannular biopsy needle (400) is shown having a closed distal end with piercing tip (402) and a lateral tissue receiving aperture (414). Marker delivery device (160) is introduced to a biopsy site through biopsy needle (400), which may be the same needle (400) used to collect a tissue sample from the biopsy site. Biopsy needle (400) may be of the type used with single insertion, multiple sample vacuum assisted biopsy devices. Several such biopsy devices are disclosed in the various patents and patent applications that have been referred to herein, though other biopsy devices may be used.

FIG. 4 shows the distal end of marker delivery device (160) disposed within needle (400). Needle (400) may be positioned in tissue, and a biopsy sample may be obtained through lateral aperture (414), thereby providing a biopsy cavity adjacent lateral aperture (414). Then, after the tissue sample has been obtained and transferred proximally through needle (400), and without removing needle (400) from the patient's tissue, marker delivery device (160) is inserted into a proximal opening in needle (400). In FIG. 4, needle (400) and marker delivery device (160) are positioned such that opening (164) of cannula (162) and lateral aperture (414) of needle (400) are substantially aligned axially and circumferentially. Then, with marker delivery device (160) and needle (400) so positioned at the biopsy site, push rod (168) is advanced to deploy marker (300) up ramp surface (212), through opening (164), and then through lateral aperture (414), into the biopsy cavity.

### III. Exemplary Biopsy Site Markers for Limited Migration

In some examples it may be desirable to include certain features within a marker similar to marker (100) to reduce a risk of the marker to migrate when placed within tissue. For instance, some markers may be prone to migration after placement of a biopsy site due to movement of tissue in the intervening time between marker placement and subsequent follow-up procedures. As a result, such markers may introduce challenges with identifying the biopsy site during subsequent follow-up procedures. Accordingly, it may be desirable to incorporate features into a marker similar to marker (100) to maintain the marker in a fixed position within tissue over time. Although several examples are described herein that incorporate the features outlined above, it should be understood that various alternative combinations can be used without departing from the basic principles described herein.

### A. Exemplary Biopsy Site Marker with Extendable Sides

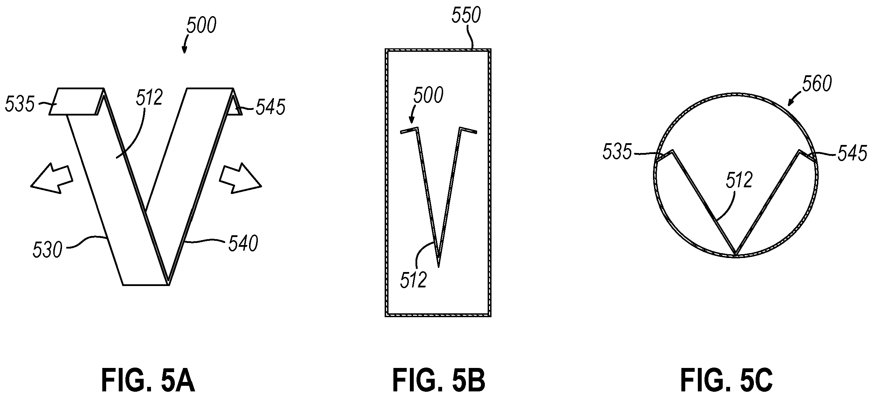

FIGS. 5A-5C show an exemplary marker (500) that is generally configured to expand outwardly to thereby anchor marker (500) within tissue. Unless otherwise explicitly noted herein, marker (500) is substantially similar to marker (100) described above. For instance, like with marker (100), marker (500) of the present example includes a marker element (512). In some examples, marker element (512) can be substantially similar to marker element (12) described above. Thus, marker element (512) can be generally configured as non-bioabsorbable and radiopaque and/or echogenic to enhance visualization over time. Marker element (512) is used with a carrier such as carrier (120) discussed above.

Marker element (512) of the present example is composed of nitinol. In certain embodiments, marker element (512) is composed of a nitinol mesh, wire, or stint like material. Additionally, in some examples marker element (512) can be configured to promote tissue ingrowth. For instance, in some examples, marker element (512) is composed of a nitinol mesh wherein the holes of the mesh are large enough so the tissue can grow through the holes, which fixes element marker (512) to the tissue. Similarly, marker element (512) can comprise a variety of materials such as metal, hard plastic, and/or etc.

Marker element (512) of the present example is configured to have a general "V" shape. In particular, marker element (512) of the present example comprises first arm (530) (also may be referred to as a "first portion") and second arm (540) (also may be referred to as a "second portion") joined along an edge and extending out, forming the "V" shape. Further, a first tine (535) is joined to first arm (530) to extend outwardly from first arm (530) and a second tine (545) is joined to second arm (540) to extend outwardly from second arm (540). In some examples, tines (535, 545) may also be referred to as an anchor, a ground, and/or etc. Each of arms (530, 540) and tines (535, 545) may have a thickness and width as required to maintain the shape of marker element (512). In some examples, tines (535, 545) are configured to have rough or sharp edges to enhance tines (535, 545) ability to grip surfaces such as tissue.

Marker element (512) is configured to be compressible so that the acute angle between first arm (530) and second arm (540) will become smaller when a force is applied inwardly to first arm (530) and/or second arm (540). Additionally, marker element (512) is resiliently biased such that marker element (512) is configured to transition from a compressed state (FIG. 5B) to an extended state (FIG. 5C) when the force is reduced or removed from first arm (530) and/or second arm (540). In other words, first arm (530) and second arm (540) are generally configured to be displaced outwardly when deployed at a biopsy site. As shown in FIG. 5B, in some examples, marker (500) can be initially confined within an outer cannula (550) or other means of confinement such as a tube, a carrier similar to carrier (120), or other structure prior to deployment to the biopsy site. While located in outer cannula (550) marker element (512) is in a compressed state. Upon deployment from outer cannula (550) into the biopsy site, marker element (512) returns to an extended state, wherein first arm (530) and second arm (540) extend away from each other. When marker element (512) returns to an extended state, first tine (535) and second tine (545) press into biopsy site wall (560), securing the marker element (512) to biopsy site wall (560). As a consequence, the widened "V" shape of marker element (512) caused by the increased distance between first arm (530) and second arm (540) can act as an anchor to hold marker element (512) at a given position within tissue. Additionally, the movement of marker element (512) from the compressed state to the extended state can cause marker element (512) to anchor to tissue. The rough or sharp edges of tines (535, 545) improves tines (535, 545) ability to grip the biopsy site wall (560). Additionally, the edges of arms (530, 540) may also include rough or sharp edges to improve the gripping ability of marker (500).

Although marker element (512) of the present example is configured with a "V" shape, it should be understood that various alternative shapes may be used. For instance, in one alternative configuration of marker element (512), the shape of marker element (512) may be of an irregular shape referred to as a "target." In this configuration, marker element (512) may be formed of one or more wires defining two opposing tightly wound coils. The two tightly wound coils may be aligned along a common axis with a loose coil joining the two opposing tightly wound coils together. The tightly wound coils may be formed by the one or more wires being wound in circular or oval-shaped coils positioned closely together. The loose coil may be a section of wire joining the two coils. The loose coil itself may include one or more coils as it extends from one tightly wound coil to the other. In this configuration, when marker element (512) is viewed from the side (e.g., angle of view parallel to the common axis), the tightly wound coils may appear as a circular or oval-shaped outline of the "target." Meanwhile, the loose coil may appear as an S-shaped pattern extending from one side of the circular or oval-shaped outline to the other. In some versions of the "target," the loose coil may also include one or more thick or round portions to make the center of the "target" more readily identifiable.

**In** another alternative configuration of marker element (512), the shape of marker element (512) may be of a "3D M" shape. In this configuration, marker element (512) may be formed of a flat sheet material similar to the "V" shape described above. However, unlike the "V" shape, the "3D M" shape may include two additional bends to form an M or W shape when viewed from one side. Thus, a total of three bends may be used. The three bends may together define a four discrete sections of material. In some examples, one or more sections may include one or more cut-outs, bores, or openings therein. Such a cut-out may be desirable to improve visualization from the top or bottom of the M-shaped pattern using an imaging medium such as x-ray where depth visualization may be difficult.

In yet another alternative configuration of marker element (512), the shape of marker element (512) may be of a "double loop" shape. In this configuration, marker element (512) may be formed of one or more wires. The one or more wires may define two circular or oval-shaped loops joined together. To improve visualization, the two loops may be oriented at different angles. For instance, a first loop may define a first plane and a second loop may define a second plane. In some examples, the first plane may be offset from the second plane by approximately 90 degrees. From above, this configuration may have the appearance of a cross. In other examples, the particular angle of offset may be varied such as the first plane and second plane being offset by approximately 45 degrees to have an X-shape when viewed from the top.

**In** still another alternative configuration of marker element (512), the shape of marker element (512) may be of a "funnel." In this configuration, marker element (512) may be formed of one or more wires. The one or more wires may define a helical or spiral pattern, with the diameter of the helical or spiral pattern decreasing with each successive coil, thereby forming a frustoconical-shaped pattern. When viewed from above, such a configuration may have a 2 dimensional spiral appearance.

### B. Exemplary Biopsy Site Marker with Dissolvable Carrier

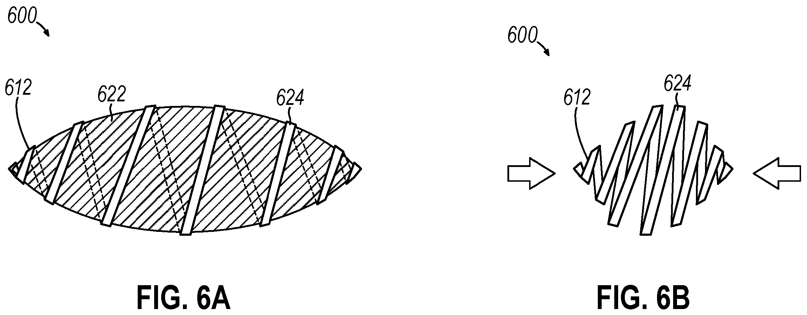

FIGS. 6A and 6B show an exemplary marker (600) that is generally configured to retract in length to thereby anchor marker (600) within tissue. Unless otherwise explicitly noted herein, marker (600) is substantially similar to marker (100) described above. For instance, like with marker (100), marker (600) of the present example includes a marker element (612). In some examples, marker element (612) can be substantially similar to marker element (12) described above. Thus, marker element (612) can be generally configured as non-bioabsorbable and radiopaque and/or echogenic to enhance visualization. In some examples, marker element (612) may be used with a carrier such as carrier (120) discussed above. Marker element (612) of the present example is composed of nitinol. In some examples, marker element (612) is composed of a nitinol mesh, wire, or stint like material. Additionally, marker element (612) can be configured to promote tissue ingrowth. For instance, marker element (612) can be composed of a nitinol mesh wherein the holes of the mesh are large enough so the tissue can grow through the holes, which fixes element marker (612) to the tissue. Similarly, marker element (612) can comprise a variety of materials such as metal, hard plastic, and/or etc.

As with marker element (12) described above, marker element (612) of the present example is configured as non-absorbable. However, unlike marker element (12), marker element (612) of the present example includes multiple a marker materials (622, 624). Marker materials (622, 624) of the present examples are generally configured to have varying material properties that effect the retraction and/or expansion thereof such that marker (600) can transition from a relatively large or extended volume to a relatively small volume. For instance, in the present example an outer marker material (622) is disposed around a structure of inner marker material (624). As will be described in greater detail below, outer marker material (622) is generally configured to dissolve over time within a patient to thereby providing a means for rapidly retracting the length of marker (600). In some examples, outer marker material (622) may also expand before dissolving, in such examples, this material property may be used to expand inner marker material (624) initially to lodge one or more anchor features of inner marker material (624) into tissue to initially anchor marker element (612) within tissue.

Marker materials (622, 624) are generally positioned in filled or layered arrangement. For instance, in some examples both marker materials (622, 624) form a prolate spheroid shape when in a dehydrated condition. In this configuration, inner marker material (624) forms a structural wound spring core that is wrapped by a filler marker material (622) also with a prolate spheroid form. Thus, marker (600) generally defines an elongate prolate spheroid configuration. In the present configuration, inner marker material (624) is shown as being centrally positioned within filler marker material (622). However, it should be understood that in other examples inner marker material (624) can have a variety of positions within filler marker material (622). Additionally, it should be understood that in other examples inner marker material (624) may be in the form of a rectangular spring, wherein both the marker material (622, 624) form a mirrored wedge, with a profile as shown in FIGS. 6A-6B.

As described above, filler marker material (622) is generally absorbable such that filler marker material (622) is configured to degrade over time within a patient. Filler marker material (622) in the present example is formed of any suitable bioabsorbable material such as hydrogel, collagen, and/or etc. Additionally, such materials can be configured to have varying absorption properties. For instance, in some examples, filler marker material (622) can be configured to absorb relatively slowly over time leading to slow contraction of inner marker material (624). Yet in other examples, filler marker material (622) can be configured to absorb relatively quickly over time leading to rapid contraction of inner marker material (624).

**In** use, marker (600) is initially placed as shown in FIG. 6A. In this state, filler marker material (622) dissolves or otherwise absorbs into tissue. As filler marker material (622) dissolves, inner marker material (624) remains and begins to expand or contract in response to movement of filler marker material (622). For instance, in some examples, filler marker material (624) may initially expand as moisture is absorbed by filler marker material (624). This expansion may initially cause an anchor portion, barb, or other structure of inner marker material (624) to be driven into tissue by filler marker material (624) expanding the structure of inner marker material (624). Upon absorption of filler marker material (624), inner marker material (624) may then retract into the space left by absorption of filler marker material (622). Compression of inner marker material (624) can continue until inner marker material (624) transitions to the compressed configuration shown in FIG. 6B.

These expansion and/or retraction properties, together with the geometry of inner marker material (624) and dissolving outer marker material (622), result in marker (600) retracting in size. In some examples, the volume of marker (600) can retract to 2/3 to 1/2 size. As a consequence, the retraction of outer marker material (622) can grip the biopsy cavity such that inner marker material (624) can act as an anchor to hold marker (600) at a given position within tissue. For instance, during retraction, folds of tissue can become trapped within the spaces defined by inner marker material (624) that were previously filled by filler marker material (622).

**In** the present example, the approximate profile of marker (600) remains generally cylindrical and/or prolate spheroid in shape. Alternatively, the shape of marker (600) after at least some hydration can be characterized as spring-shaped. Of course, various other alternative profiles can be formed using the present configuration. For instance, in other examples the initial geometry of outer marker material (622) can be varied to influence the profile of marker (600) after at least some hydration. In addition, it should be understood that the particular shape and/or size of marker (600) can change throughout the course of hydration. For instance, marker (600) can start in the position shown in FIG. 6A after initial hydration. As hydration completes, marker (600) can exhibit some additional expansion. Subsequently, filler marker material (622) can dissolve and inner marker material (624) can retract.

### C. Exemplary Biopsy Site Marker with One Directional Screw

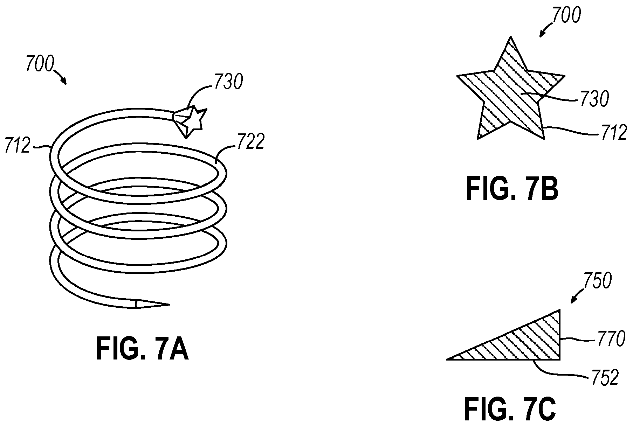

FIGS. 7A and 7B show an exemplary marker (700) that is generally configured to screw into biopsy site when deployed to thereby anchor marker (700) within tissue. Unless otherwise explicitly noted herein, marker (700) is substantially similar to marker (100) described above. For instance, like with marker (100), marker (700) of the present example includes a marker element (712). In some examples, marker element (712) can be substantially similar to marker element (12) described above. Thus, marker element (712) can be generally configured as non-bioabsorbable and radiopaque and/or echogenic to enhance visualization.

Marker element (712) is used with a carrier such as carrier (120) discussed above.

Marker element (712) of the present example is composed of nitinol. In some examples, marker element (712) is composed of a nitinol mesh, wire, or stint like material. Additionally, in some examples marker element (712) can be configured to promote tissue ingrowth. For instance, in such examples, marker element (712) is composed of a nitinol mesh wherein the holes of the mesh are large enough so the tissue can grow through the holes, which fixes element marker (712) to the tissue. In still other examples, marker element (712) can comprise a variety of materials such as metal, hard plastic, and/or etc.

As with marker element (12) described above, marker element (712) of the present example is configured to be non-absorbable. However, unlike marker element (12), marker element (712) of the present example is configured to be screwed into tissue of biopsy site and includes an angled barb or tab (730) at one end of screw marker element (712) to prevent unscrewing or backout when placed in tissue. Marker materials (722) of the present example is generally configured to have material properties that support the structure with sufficient rigidity that marker (700) can be screwed into tissue of biopsy site. Marker material (722) generally forms a helical or spiral shape to define marker element (712), as well as tab (730) at the end of the spiral form.

As best seen in FIG. 7A, the generally helical shape of marker material (722) is interrupted at one end by a tab (730). As will be described in greater detail below, tab (730) is generally configured to enhance the grip of tissue by marker element (712). Tab (730) of the present example can take on a variety of forms. For instance, as shown in FIG. 7B, tab (730) can have a generally star-shaped cross-section. Alternatively, tab (770) can have a generally triangular-shaped cross-section, as will be described in greater detail below. Regardless of the particular shape of tab (730), tab (730) is generally configured as a protrusion on an end surface of marker element (712) which extends radially outward. It should be understood that in other examples tab (730) can take on a variety of alternative shapes. For instance, in some examples, tabs (730) are rounded, squared, rectangular, and/or etc. In addition, or in the alternative, the particular shape of tab (730) can be extended to the remainder of marker element (712) such that marker element (712) has a continuous profile.

As noted above, tab (730) of marker (700) can take on a variety of forms. For instance, FIG. 7C shows a marker (750) that is substantially similar to marker (700) described above, except marker (750) includes a tab (770) having a generally triangular-shaped cross-section. It should be understood that unless otherwise explicitly stated herein, marker (750) is identical to marker (700) described above. For instance, like with marker (700), marker (750) of the present example includes an elongate marker element (752) shaped in a helical or screw-shaped pattern with a tab (770) on one end.

As similarly described above with respect to tab (730), tab (770) is generally configured to enhance the grip of tissue by marker element (752). Tab (770) of the present example can take on a variety of forms. For instance, as shown in FIG. 7C, tab (770) can have a generally triangular-shaped cross-section. Regardless of the particular shape of tab (770), tab (770) is generally configured as a protrusion on an end surface of marker element (752) which extends radially outward. It should be understood that in other examples tab (770) can take on a variety of alternative shapes. For instance, in some examples, tab (770) is rounded, squared, rectangular, and/or etc. In addition, or in the alternative, the particular shape of tab (770) can be extended to the remainder of marker element (752) such that marker element (752) has a continuous profile.

### D. Exemplary Biopsy Site Marker with One Directional Coil

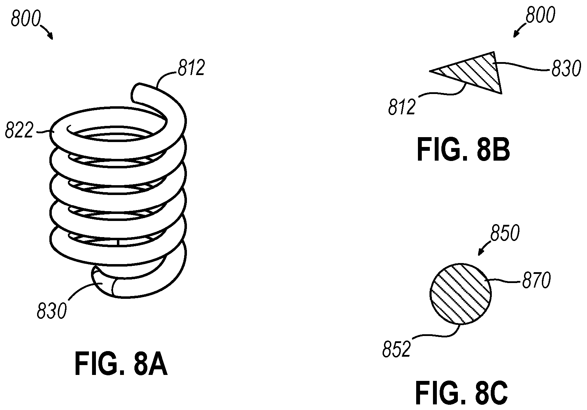

FIGS. 8A and 8B show an exemplary marker (800) that is generally configured to screw into biopsy site when deployed to thereby anchor marker (800) within tissue. Unless otherwise explicitly noted herein, marker (800) is substantially similar to marker (100) described above. For instance, like with marker (100), marker (800) of the present example includes a marker element (812). In some examples, marker element (812) can be substantially similar to marker element (12) described above. Thus, marker element (812) can be generally configured as non-bioabsorbable and radiopaque and/or echogenic to enhance visualization.

Marker element (812) is used with a carrier such as carrier (120) discussed above.

Marker element (812) of the present example is composed of nitinol. In some examples, marker element (812) is composed of a nitinol mesh, wire, or stint like material. In other examples, marker element (812) can be configured to promote tissue ingrowth. For instance, in such examples, marker element (812) is composed of a nitinol mesh wherein the holes of the mesh are large enough so the tissue can grow through the holes, which fixes element marker (812) to the tissue. In still other examples, marker element (812) can comprise a variety of materials such as metal, hard plastic, and/or etc.

As with marker element (12) described above, marker element (812) of the present example is configured to be non-absorbable. However, unlike marker element (12), marker element (812) of the present example is configured to be screwed into tissue of biopsy site and includes an undercut tab (830) at one end of screw marker element (812) to prevent unscrewing or backout when placed in tissue. Marker materials (822) of the present examples are generally configured to have material properties that support the structure with sufficient rigidity that marker (800) can be screwed into tissue of biopsy site. Marker material (822) generally forms a helical or coil shape to define marker element (812), as well as tab (830) at the end of the spiral form. In the present example marker element (812) cross-section can be circular or triangular as best shown in FIG. 8B. It should be understood that in other examples marker element (812) cross-section can take on a variety of alternative shapes.

As best seen in FIG. 8A, the generally helical shape of marker material (822) is interrupted at one end by an undercut tab (830). Tab (830) is generally configured to enhance the grip of tissue by marker element (812). As seen in FIG. 8B, tab (830) of the present example has a generally triangular cross-section. In other examples, tab (830) can take on a variety of forms such as conical or pointed, as will be described in greater detail below. Regardless of the particular profile, tab (830) is generally angled from the helical axis defined by marker element (812) on an end surface of marker element (812). It should be understood that in other examples tab (830) can take on a variety of alternative shapes. For instance, in some examples, tabs (830) are rounded, squared, rectangular, and/or etc.

As noted above, tab (830) of marker (800) can take on a variety of forms. For instance, FIG. 8C shows a marker (850) that is substantially similar to marker (800) described above, except marker (850) includes a tab (870) having a conical or circular cross-section. It should be understood that unless otherwise explicitly stated herein, marker (850) is identical to marker (800) described above. For instance, like with marker (800), marker (850) of the present example includes an elongate marker element (852) shaped in a helical or screw-shaped pattern with a tab (870) on one end.

As similarly described above with respect to tab (830), tab (870) is generally configured to enhance the grip of tissue by marker element (852). Tab (870) of the present example can take on a variety of forms. For instance, as shown in FIG. 8C, tab (870) can have a generally circular cross-section to form a conical pointed tip. Regardless of the particular shape of tab (870), tab (870) is generally configured as a protrusion on an end surface of marker element (852) which extends radially outward. It should be understood that in other examples tab (870) can take on a variety of alternative shapes. For instance, in some examples, tab (870) is rounded, squared, rectangular, and/or etc. In addition, or in the alternative, the particular shape of tab (870) can be extended to the remainder of marker element (852) such that marker element (852) has a continuous profile.

### E. Exemplary Biopsy Site Marker with Serrated Screw

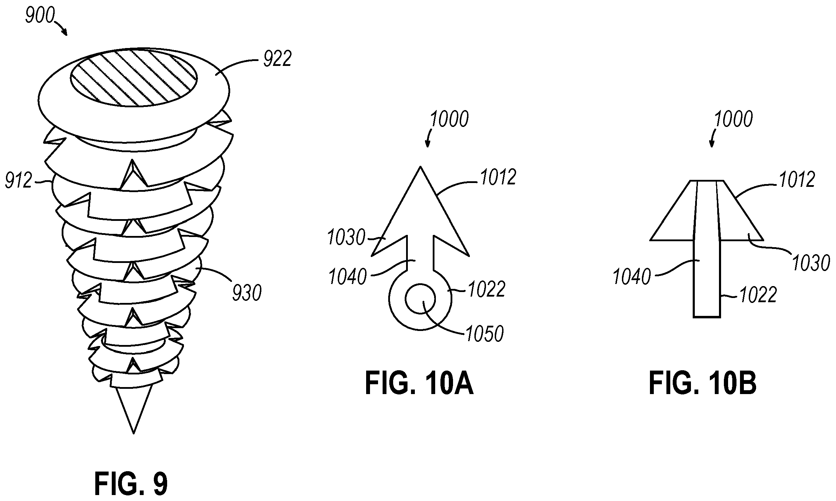

FIG. 9 shows an exemplary marker (900) that is generally configured to screw into biopsy site when deployed to thereby anchor marker (900) within tissue. Unless otherwise explicitly noted herein, marker (900) is substantially similar to marker (100) described above. For instance, like with marker (100), marker (900) of the present example includes a marker element (912). In some examples, marker element (912) can be substantially similar to marker element (12) described above. Thus, marker element (912) can be generally configured as non-bioabsorbable and radiopaque and/or echogenic to enhance visualization over time. Although not shown, it should be understood that marker element (912) can optionally be used with a carrier such as carrier (120) discussed above.

Marker element (912) of the present example is composed of nitinol. In some examples, marker element (912) is composed of a nitinol mesh, wire, or stint like material. Additionally, marker element (912) can be configured to promote tissue ingrowth. For instance, in such examples, marker element (912) is composed of a nitinol mesh wherein the holes of the mesh are large enough so the tissue can grow through the holes, which fixes element marker (912) to the tissue. Similarly, marker element (912) can comprise a variety of materials such as metal, hard plastic, and/or etc.

As with marker element (12) described above, marker element (912) of the present example is non-absorbable. However, unlike marker element (12), marker element (912) of the present example is configured to be screwed into tissue of biopsy site and includes serrated edge (930) along a helical edge of the marker element (912) to prevent unscrewing or backout when placed in tissue. Marker materials (922) of the present examples are generally configured to have material properties that support the structure with sufficient rigidity that marker (900) can be screwed into tissue of biopsy site. Marker material (922) generally forms a helical cone shape to define marker element (912), as well as serrated edge (930) along the edges of the conical form as shown. In the present example marker element (912) height and width can be any dimensions suitable for the biopsy procedure. In the present example serrated edge (930) can be non-uniform and along a single edge. It should be understood that in other examples serrated edge (930) can take on a variety of alternative shapes, such as uniform serrations or multiple edges.

As seen in FIG. 9, the generally conical shape of marker material (922) is abutted on the conical surface by serrated edge (930). As will be described in greater detail below, edge (930) is generally configured to enhance the grip of tissue by carrier (912). Edge (930) of the present example is formed as generally pitched screw thread rising along the conical surface of marker element (912). It should be understood that in other examples edge (930) can take on a variety of alternative shapes. For instance, in some examples, tabs (930) are rounded, squared, rectangular, and/or etc.

### F. Exemplary Biopsy Site Marker with Barbs

FIGS. 10A and 10B show an exemplary marker (1000) that is generally configured to screw into biopsy site when deployed to thereby anchor marker (1000) within tissue. Unless otherwise explicitly noted herein, marker (1000) is substantially similar to marker (100) described above. For instance, like with marker (100), marker (1000) of the present example includes a marker element (1012). In some examples, marker element (1012) can be substantially similar to marker element (12) described above. Thus, marker element (1012) can be generally configured as non-bioabsorbable and radiopaque and/or echogenic to enhance visualization. Although not shown, it should be understood that in some example, marker element (1012) may be used with a carrier such as carrier (120) discussed above.

Marker element (1012) of the present example is composed of nitinol. In some examples, marker element (1012) is composed of a nitinol mesh, wire, or stint like material. Additionally, marker element (1012) can be configured to promote tissue ingrowth. For instance, in such examples, marker element (1012) is composed of a nitinol mesh wherein the holes of the mesh are large enough so the tissue can grow through the holes, which fixes element marker (1012) to the tissue. In still other examples, marker element (1012) can comprise a variety of materials such as metal, hard plastic, and/or etc.

As with marker element (12) described above, marker element (1012) of the present example is configured to prevent absorption into a patient after placement of marker (1000). However, unlike marker element (12), marker element (1012) of the present example is configured to be attached to tissue of biopsy site and includes one or more barbs (1030) at one end of marker element (1012) to prevent migration of marker element (1012) when placed in tissue. Marker materials (1022) of the present examples are generally configured to have material properties that support the structure with sufficient rigidity that marker (1000) can prevent migration of the marker element (1012) from the biopsy site. Marker material (1022) generally forms a shaft (1040), an engagement body (1050) at a proximal end, and one or more barbs (1030) at a distal end. In the present example shaft (1040) can be circular or triangular. It should be understood that in other examples shaft (1040) can take on a variety of alternative shapes.

As best seen in FIG. 10A, the generally elongated shape of marker material (1022) is interrupted at a distal end by one or more barbs (1030). As will be described in greater detail below, barbs (1030) are generally configured to enhance the grip of tissue by marker element (1012). Barbs (1030) of the present example are formed as generally pointed tips projecting in a direction from an end surface of marker element (1012) so as to engage the tissue of the biopsy site. Further, the generally elongated shape of marker material (1022) is interrupted at a proximal end by an engagement body (1050) configured to engage with a marker delivery probe.

The particular barbed configuration of marker element (1012) can take on a variety of forms. For instance, as shown in FIG. 10B, marker element (1012) can alternatively comprise multiple barbs (1030) in a circular formation. It should be understood that in other examples barbs (1030) can take on a variety of alternative shapes that would be well known to one skilled in the art.

### G. Exemplary Biopsy Site Marker with Coiled Hooks

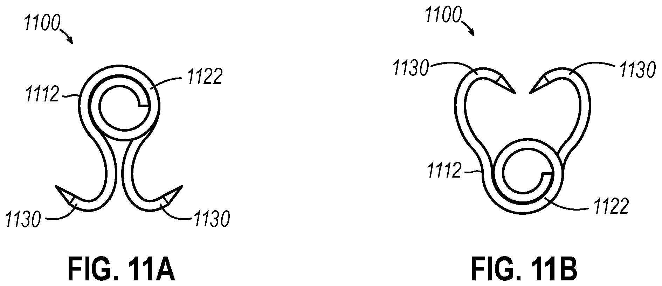

FIGS. 11A and 11B show an exemplary marker (1100) that is generally configured to attach to biopsy site when deployed to thereby anchor marker (1100) within tissue. Unless otherwise explicitly noted herein, marker (1100) is substantially similar to marker (100) described above. For instance, like with marker (100), marker (1100) of the present example includes a marker element (1112). In some examples, marker element (1112) can be substantially similar to marker element (12) described above. Thus, marker element (1112) can be generally configured as non-bioabsorbable and radiopaque and/or echogenic to enhance visualization over time. In certain examples, marker element (1112) may be used with a carrier such as carrier (120) discussed above. Marker element (1112) of the present example is composed of nitinol. In some examples, marker element (1112) is composed of a nitinol mesh, wire, or stint like material. Additionally, marker element (1112) can be configured to promote tissue ingrowth. In some examples, marker element (1112) is composed of a nitinol mesh wherein the holes of the mesh are large enough so the tissue can grow through the holes, which fixes element marker (1112) to the tissue. Similarly, marker element (1112) can comprise a variety of materials such as metal, hard plastic, and/or etc.

As with marker element (12) described above, marker element (1112) of the present example is non-absorbable. However, unlike marker element (12), marker element (1112) of the present example is configured to deploy into tissue of biopsy site and hooks (1130) which uncoil from marker element (1112) to prevent migration of marker element (1112) when placed in tissue. Marker materials (1122) of the present examples are generally configured to have material properties that support the structure with sufficient rigidity while allowing for uncoiling of hooks (1130). Marker material (1122) generally forms a center body to define marker element (1112), as well as hooks (1130) extending from marker element (1112).

As best seen in FIG. 11A, the center body of marker material (1122) included one or more hooks (1130) coiled around the center body before marker element (1112) is deployed to biopsy site. As will be described in greater detail below, hooks (1130) are generally configured to enhance the grip of tissue by marker element (1112). Hooks (1130) of the present example are formed as generally curved protrusions extending from the surface of marker element (1112) which uncoil from the pre-deployment position when placed in the biopsy site as shown in FIG. 11B. It should be understood that in other examples hooks (1130) can take on a variety of alternative shapes.

### H. Exemplary Biopsy Site Marker with Deployable Wings

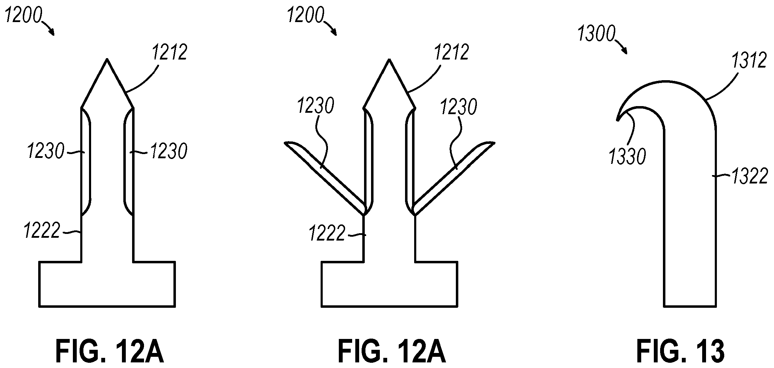

FIGS. 12A and 12B show an exemplary marker (1200) that is generally configured to attach to biopsy site when deployed to thereby anchor marker (1200) within tissue. Unless otherwise explicitly noted herein, marker (1200) is substantially similar to marker (100) described above. For instance, like with marker (100), marker (1200) of the present example includes a marker element (1212). For instance, in some examples, marker element (1212) can be substantially similar to marker element (12) described above. Thus, marker element (1212) can be generally configured as non-bioabsorbable and radiopaque and/or echogenic to enhance visualization over time. In certain examples, marker element (1212) may be used with a carrier such as carrier (120) discussed above. Marker element (1212) of the present example is composed of nitinol. In some examples, marker element (1212) is composed of a nitinol mesh, wire, or stint like material. Additionally, marker element (1212) can be configured to promote tissue ingrowth. In some examples, marker element (1212) is composed of a nitinol mesh wherein the holes of the mesh are large enough so the tissue can grow through the holes, which fixes element marker (1212) to the tissue. Similarly, marker element (1212) can comprise a variety of materials such as metal, hard plastic, and/or etc.

As with marker element (12) described above, marker element (1212) of the present example is configured to prevent absorption into a patient after placement of marker (1200). However, unlike marker element (12), marker element (1212) of the present example is configured to deploy into tissue of biopsy site and wings (1230) which open from marker element (1212) to prevent migration of marker element (1212) when placed in tissue. Marker materials (1222) of the present examples are generally configured to have material properties that support the structure with sufficient rigidity while allowing for opening of wings (1230). Marker material (1222) generally forms a center body to define marker element (1212), as well as wings (1230) extending from marker element (1212).

As best seen in FIG. 12A, the center body of marker material (1222) included one or more wings (1230) closed against the center body before marker element (1212) is deployed to biopsy site. As will be described in greater detail below, wings (1230) are generally configured to enhance the grip of tissue by marker element (1212). Wings (1230) of the present example are formed as generally elongated protrusions extending from the surface of marker element (1212) which open from the pre-deployment position when placed in the biopsy site as shown in FIG. 12B. It should be understood that in other examples wings (1230) can take on a variety of alternative shapes.

### I. Exemplary Biopsy Site Marker with Hook

FIG. 13 shows an exemplary marker (1300) that is generally configured to attach to biopsy site when deployed to thereby anchor marker (1300) within tissue. Unless otherwise explicitly noted herein, marker (1300) is substantially similar to marker (100) described above. For instance, like with marker (100), marker (1300) of the present example includes a marker element (20). In some examples, marker element (1312) can be substantially similar to marker element (12) described above. Thus, marker element (1312) can be generally configured as non-bioabsorbable and radiopaque and/or echogenic to enhance visualization. In certain examples, marker element (1312) may be used with a carrier such as carrier (120) discussed above. Marker element (1312) of the present example is composed of nitinol. In some examples, marker element (1312) is composed of a nitinol mesh, wire, or stint like material. Additionally, marker element (1312) can be configured to promote tissue ingrowth. In some examples, marker element (1312) is composed of a nitinol mesh wherein the holes of the mesh are large enough so the tissue can grow through the holes, which fixes marker element (1312) to the tissue. Similarly, marker element (1312) can comprise a variety of materials such as metal, hard plastic, and/or etc.

As with marker element (12) described above, marker element (1312) of the present example is configured non-absorbable. However, unlike marker element (12), marker element (1312) of the present example is configured to deploy into tissue of biopsy site and a hook (1330) extending from marker element (1312) to prevent migration of marker element (1312) when placed in tissue. Marker material (1322) of the present examples is generally configured to have material properties that support the structure with sufficient rigidity while allowing for gripping of hook (1330). Marker material (1322) generally forms an elongated body to define marker element (1312), as well as wings (1330) extending from marker element (1312). In still other examples, marker element (1312) of varying shapes and/or materials can be used. Although the present example is shown and described as marker element (1312) itself forming gripping hook (1330), it should be understood that in other examples gripping hook (1330) can be formed of structures similar to carrier (120) described above. Thus, in such examples, gripping hook (1330) and/or the entire exterior of marker (1300) can be comprised of hydrogel, collagen, and/or etc.

As seen in FIG. 13, the center body of marker material (1322) includes a hook (1330) extending from marker element (1312). As will be described in greater detail below, hook (1330) is generally configured to enhance the grip of tissue by marker element (1312). Hook (1330) of the present example is formed as a generally elongated protrusions extending from the surface of marker element (1312) which provides and gripping tip. It should be understood that in other examples hook (1330) can take on a variety of alternative shapes.

### J. Exemplary Biopsy Site Marker with Pad Form

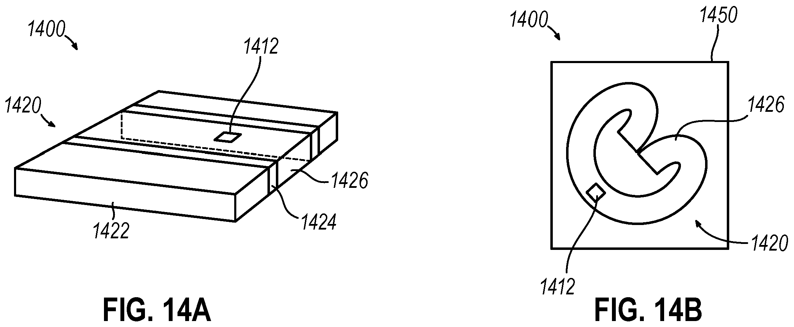

FIGS. 14A and 14B show an exemplary marker (1400) that is generally configured to rapidly unroll to thereby anchor marker (1400) within tissue. Unless otherwise explicitly noted herein, marker (1400) is substantially similar to marker (100) described above. For instance, like with marker (100), marker (1400) of the present example includes a marker element (1412). In some examples, marker element (1412) can be substantially similar to marker element (12) described above. Thus, marker element (1412) can be generally configured as non-bioabsorbable and radiopaque and/or echogenic to enhance visualization. Marker element (1412) of the present example is composed of nitinol. In some examples, marker element (1412) is composed of a nitinol mesh, wire, or stint like material. Additionally, marker element (1412) can be configured to promote tissue ingrowth. In some examples, marker element (1412) is composed of a nitinol mesh wherein the holes of the mesh are large enough so the tissue can grow through the holes, which fixes element marker (1412) to the tissue. Similarly, marker element (1412) can comprise a variety of materials such as metal, hard plastic, and/or etc.

Marker element (1412) can also take on a wide variety of shapes, and/or sizes. For instance, in some examples, marker element (1412) can have the shape of marker element (12) shown in FIG. 1 (e.g., a stylized "E" shape). In other examples, marker element (1412) can have a helical wire-shape. In yet other examples, marker element (1412) can have an irregular shape defining a plurality of reflective surfaces. In still other examples, marker element (1412) can be shaped as curved clip. In still other examples, multiple marker elements (1412) of varying shapes and/or materials can be used. Of course, still other configurations for marker element (1412) may be apparent to those of ordinary skill in the art in view of the teachings herein.

As with carrier (120) described above, carrier (1420) of the present example is configured for absorption into a patient after placement of marker (1400). However, unlike carrier (120), carrier (1420) of the present example includes multiple marker materials (1422, 1424, 1426). Marker materials (1422, 1424, 1426) of the present examples are generally configured to have varying material properties that effect the material strength and time required for degradation thereof such that marker (1400) can unroll from a relatively small volume to a relatively large or extended volume. For instance, in the present example an outer marker material (1422) is disposed on outside edges of carrier (1420). A middle marker material (1424) lies inside of outer marker material (1422). Finally, an inner marker material (1426) lies along the center of carrier (1420). As will be described in greater detail below, marker materials (1422, 1424, 1426) are generally configured to be flexible enough to allow the carrier (1420) to be in a pad form to be rolled prior to deployment, as shown in FIG. 14B, thereby providing a means for rapidly unrolling the length of marker (1400). In some examples, as that shown in FIGS. 14A and 14B, the marker materials (1422, 1424, 1426) are collagen or hydrogel. Additionally, there may be as many marker materials (1422, 1424, 1426) as is suitable for the carrier (1420).

Marker materials (1422, 1424, 1426) are generally positioned in a layered arrangement, moving from an outer edge of carrier (1420) to a center. For instance, in some examples marker materials (1422, 1424, 1426) form a rolled pad when in a dehydrated condition before deployment. It should be understood that in other examples inner marker material (1426) can have a variety of positions within carrier (1420). Additionally, it should be understood that in other examples inner marker material (1426) may be in the form of a square surrounded by the middle marker material (1424), or wherein the carrier is rectangular are square in shape.

As best seen in FIG. 14A, marker element (1412) of the present example is generally centered or substantially centered within inner marker material (1426). This central configuration can be desirable so that marker element (1412) remains centered within a biopsy site as marker materials (1422, 1424, 1426) degrade or absorb into tissue. However, it should be understood that marker element (1412) can be placed in various alternative positions either within inner marker material (1426) or partially within inner marker material (1426) and partially within middle marker material (1424) or outer marker material (1422).

FIG. 14B shows marker (1400) in a deployed state after marker (1400) is positioned within tissue. To reach this state, marker (1400) can be rolled to the position shown in FIG. 14B from a natural state shown in FIG. 14A. In this state, carrier (1420) can unroll towards the natural state shown in FIG. 14A as marker materials (1422, 1424, 1426) hydrate. In some examples, marker materials (1422, 1424, 1426) can be configured to affect the rate of unrolling and/or the shape during unrolling. For instance, inner marker material (1426) can be configured to absorb moisture relatively quickly and thereby exhibit relatively high degradation or quick expansion. This property can be used to drive unrolling of marker (1400). Meanwhile, while middle marker material (1424) can be configured to expand relatively slowly to provide structural stability and/or durability. Finally, outer marker material (1422) exhibits can be configured to absorb fluid rapidly to also drive unrolling of marker (1400). These expansion properties, together with the geometry of carrier (1420) pad form, result in marker (1400) rapidly expanding in size. In some examples, the surface area of marker (1400) can expand to 1/2 to 2/3 size. As a consequence, the rapid expansion of marker (1400) can grip the biopsy cavity such that carrier (1420) can act as an anchor to hold marker (1400) at a given position within tissue.

In the present example, the approximate profile of marker (1400) remains generally rectangular in shape upon deployment, but moves from the rolled to unrolled configuration. Of course, various other alternative profiles can be formed using the present configuration. For instance, in other examples the initial geometry of marker (1400) profile can be varied to influence the expansion of marker (1400) after unrolling and at least some hydration. In addition, it should be understood that the particular shape and/or size of marker (1400) can change throughout the course of hydration. For instance, marker (1400) can start in the position shown in FIG. 14B after initial deployment. As hydration completes, marker (1400) can form a shape approximately corresponding to the shape shown in FIG. 14A or another shape between the shapes shown in FIGS. 14A and 14B. Subsequently, inner marker material (1426) can expand further before absorbing into tissue.

### V. Conclusion

Having shown and described various embodiments of the present invention, further adaptations of the methods and systems described herein may be accomplished by appropriate modifications by one of ordinary skill in the art. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention is defined by the claims.

## Claims

1. A biopsy site marker, the biopsy site marker comprising :
a marker element (612, 712, 1112) having a resilient portion, the resilient portion being configured to transition the marker element between a pre-deployment state and a deployment state, the marker element being configured to grip tissue when in the deployment state; **characterized by**
a bioabsorbable carrier (120), the marker element (612, 712, 1112) forming a substantially helical pattern, the substantially helical pattern being disposed within a portion of the carrier (120).

2. The biopsy site marker of claim 1, the marker element (1112) defining a first arm and a second arm that together form a V-shaped pattern, the resilient portion being configured to flex the first arm and the second arm outwardly when transitioning from the pre-deployment state to the deployment state.

3. The biopsy site marker of claims 1 or 2, the resilient portion being incorporated into the substantially helical pattern of the marker element (612, 712, 1112), the resilient portion being configured to retract the length of the substantially helical pattern of the marker element (612, 712, 1112) when transitioning from the pre-deployment state to the deployment state.

4. The biopsy site marker of any of claim 1, wherein the carrier (120) surrounds the marker element (612, 712, 1112) such that the carrier (120) is configured to maintain the resilient portion in the pre-deployment state.

5. The biopsy site marker of claim 1, wherein the marker element (612, 712, 1112) includes a tab (730, 770), wherein the tab is configured to secure the marker element (612, 712, 1112) to tissue.

6. The biopsy site marker of claim 5, wherein the tab (770) defines a triangular-shaped cross-section.

7. The biopsy site marker of claim 5, wherein the tab (730) defines a star-shaped cross-section.

8. The biopsy site marker of claim 5, wherein the tab defines a circular-shaped cross-section.

## Patentansprüche

1. Biopsiestellenmarker, wobei der Biopsiestellenmarker umfasst:
ein Markerelement (612, 712, 1112) mit einem federelastischen Abschnitt, wobei der federelastische Abschnitt dazu ausgebildet ist, das Markerelement zwischen einem nicht freigesetzten Zustand und einem freigesetzten Zustand zu überführen, wobei das Markerelement dazu ausgebildet ist, Gewebe zu greifen, wenn es sich in dem freigesetzten Zustand befindet; **dadurch gekennzeichnet, dass**
einen bioabsorbierbaren Träger (120), wobei das Markerelement (612, 712, 1112) ein im Wesentlichen helikales Muster bildet, wobei das im Wesentlichen helikale Muster innerhalb eines Abschnitts des Trägers (120) angeordnet ist.

2. Biopsiestellenmarker nach Anspruch 1,
wobei das Markerelement (1112) einen ersten Arm und einen zweiten Arm definiert, die zusammen ein V-förmiges Muster bilden, wobei der federelastische Abschnitt dazu ausgebildet ist, den ersten Arm und den zweiten Arm beim Übergehen von dem nicht freigesetzten Zustand in den freigesetzten Zustand nach außen zu biegen.

3. Biopsiestellenmarker nach den Ansprüchen 1 oder 2, wobei der federelastische Abschnitt in das im Wesentlichen helikale Muster des Markerelements (612, 712, 1112) integriert ist, wobei der federelastische Abschnitt dazu ausgebildet ist, die Länge des im Wesentlichen helikalen Musters des Markerelements (612, 712, 1112) beim Übergehen von dem nicht freigesetzten Zustand in den freigesetzten Zustand einzuziehen.

4. Biopsiestellenmarker nach einem des Anspruchs 1, wobei der Träger (120) das Markerelement (612, 712, 1112) so umgibt, dass der Träger (120) dazu ausgebildet ist, den federelastischen Abschnitt in dem nicht freigesetzten Zustand zu halten.

5. Biopsiestellenmarker nach Anspruch 1, wobei das Markerelement (612, 712, 1112) eine Lasche (730, 770) einschließt,
wobei die Lasche dazu ausgebildet ist, das Markerelement (612, 712, 1112) an Gewebe zu sichern.

6. Biopsiestellenmarker nach Anspruch 5, wobei die Lasche (770) einen dreieckförmigen Querschnitt definiert.

7. Biopsiestellenmarker nach Anspruch 5, wobei die Lasche (730) einen sternförmigen Querschnitt definiert.

8. Biopsiestellenmarker nach Anspruch 5, wobei die Lasche einen kreisförmigen Querschnitt definiert.

## Revendications

1. Marqueur de site de biopsie, le marqueur de site de biopsie comprenant :
un élément marqueur (612, 712, 1112) ayant une partie élastique, la partie élastique étant configurée pour faire passer l'élément marqueur entre un état de pré-déploiement et un état de déploiement, l'élément marqueur étant configuré pour agripper le tissu lorsqu'il est dans l'état de déploiement ; **caractérisé par**
un support bioabsorbable (120), l'élément marqueur (612, 712, 1112) formant une configuration sensiblement hélicoïdale, la configuration sensiblement hélicoïdale étant disposée à l'intérieur d'une portion du support (120).

2. Marqueur de site de biopsie selon la revendication 1,
l'élément marqueur (1112) définissant un premier bras et un second bras qui ensemble forment une configuration en V, la partie élastique étant configurée pour faire fléchir le premier bras et le second bras vers l'extérieur lors du passage de l'élément marqueur de l'état de pré-déploiement à l'état de déploiement.

3. Marqueur de site de biopsie selon les revendications 1 ou 2, la partie élastique étant incorporée dans la configuration sensiblement hélicoïdale de l'élément marqueur (612, 712, 1112), la partie élastique étant configurée pour rétracter la longueur de la configuration sensiblement hélicoïdale de l'élément marqueur (612, 712, 1112) lors du passage de l'élément marqueur de l'état de pré-déploiement à l'état de déploiement.

4. Marqueur de site de biopsie selon n'importe laquelle de la revendication 1, dans lequel le support (120) entoure l'élément marqueur (612, 712, 1112) de telle sorte que le support (120) soit configuré de manière à maintenir la partie élastique dans l'état de pré-déploiement.

5. Marqueur de site de biopsie selon la revendication 1, dans lequel l'élément marqueur (612, 712, 1112) comprend une patte (730, 770),
dans lequel la patte est configurée pour fixer l'élément marqueur (612, 712, 1112) au tissu.

6. Marqueur de site de biopsie selon la revendication 5, dans lequel la patte (770) définit une section transversale de forme triangulaire.

7. Marqueur de site de biopsie selon la revendication 5, dans lequel la patte (730) définit une section transversale de forme étoilée.

8. Marqueur de site de biopsie selon la revendication 5, dans lequel la patte définit une section transversale de forme circulaire.
